# DEMANDE DE BREVET EUROPEEN

(11) **EP 1 741 363 A1**
(43) Date de publication de la demande: **10.01.2007**
(21) Numéro de dépôt: 06300780.1
(22) Date de dépôt: 07.07.2006
(51) Int. Cl.: A45D 29/00, A61K 8/00

(54) **Procédé de maquillage faisant intervenir une interaction magnétique**

(30) Priorité: 08.07.2005 FR 0552125
(71) Demandeur: L'ORÉAL, 75008 Paris (FR)
(72) Inventeur: Thevenet, Ludovic, 92340 Bourg la reine (FR)
(74) Mandataire: Tanty, François

(57) **Abrégé**

La présente invention concerne un procédé de maquillage et/ou de décor des matières kératiniques, notamment de la peau, des ongles, ou des cils, le procédé comportant les étapes consistant à :
- i) appliquer sur lesdites matières kératiniques une première couche (1 ; 11) d'une première composition (P) ou une première structure (15) présentant des propriétés magnétiques,
- ii) appliquer sur une portion au moins de la première couche ou de la première structure, une seconde couche d'une seconde composition ou une seconde structure (5 ; 9 ; 10) interagissant magnétiquement avec la première couche ou avec la première structure,
- l'une au moins des première et deuxième structures comportant des corps magnétiques dispersés dans un milieu non magnétique.

## Description

La présente invention concerne un procédé de maquillage d'un support, naturel tel que la peau, les phanères ou les lèvres, ou artificiel tel que les faux ongles, ainsi que divers kits, articles et compositions pour la mise en oeuvre de ce procédé.

US 2001/022025 divulgue un procédé de rasage comprenant l'application d'une solution comportant des particules magnétiques, le rasage s'effectuant avec un rasoir aimanté.

US 2003//007942 divulgue un dispositif pour retenir des germes de la peau comprenant l'application d'une composition comportant des particules magnétiques qui se lient aux germes puis son extraction à l'aide d'aimants.

Ces deux publications ne concernent pas des procédés de maquillage.

Des procédés de maquillage comportant l'application successive de deux produits sont connus. Le deuxième produit appliqué sur le premier produit vise par exemple à améliorer la tenue ou la brillance.

Il existe un besoin pour offrir encore de nouvelles possibilités de maquillage, par exemple pour permettre d'enlever ou de modifier aisément un maquillage et/ou créer de nouveaux effets esthétiques.

### Procédé de maquillage

Selon l'un de ses aspects, parmi d'autres, l'invention a pour objet un procédé de maquillage des matières kératiniques, notamment de la peau, des ongles, ou des cils, comportant les étapes consistant à :
- i) appliquer sur lesdites matières kératiniques une première couche d'une première composition ou une première structure présentant des propriétés magnétiques,
- ii) appliquer sur une portion au moins de la première couche ou de la première structure, une seconde couche d'une seconde composition ou une seconde structure interagissant magnétiquement avec la première couche ou avec la première structure,

- l'une au moins des première et deuxième structures comportant des corps magnétiques dispersés dans un milieu non magnétique.

Le terme « maquillage » doit être compris au sens large et couvre l'application ou formation d'un décor sur le corps ou le visage.

Par « dispersés » on entend « répartis » dans un milieu non magnétique de la composition ou de la structure. Quand la composition ou la structure est destinée à être appliquée directement sur les matières kératiniques, le milieu est physiologiquement acceptable. Un aimant monolithique réalisé par frittage n'est pas un exemple de particules magnétiques dispersées car les particules magnétiques sont agglomérées.

Par « propriétés magnétiques », il faut comprendre une susceptibilité magnétique non nulle, suffisante pour que les effets liés à l'interaction magnétique, par exemple visuels et/ou d'attraction, soient perceptibles par l'utilisateur lors de la mise en oeuvre du procédé.

Par « interaction magnétique », on entend une interaction entre un corps magnétique, c'est-à-dire un corps aimanté ou aimantable et un autre corps générant un champ magnétique. Cette interaction peut créer des forces d'attraction ou de répulsion entre les deux corps et/ou amener l'un au moins des corps à modifier son orientation et/ou à se déplacer relativement à l'autre corps.

L'interaction magnétique due par exemple à une aimantation permanente de la première couche ou première structure et/ou de la deuxième couche ou deuxième structure peut assurer le maintien réversible de la deuxième couche ou structure sur la première couche ou structure. En variante, ce maintien peut être assuré au moins partiellement autrement que par une interaction magnétique, notamment par une adhérence liée à l'utilisation d'un adhésif par exemple. Cet adhésif peut appartenir à la deuxième couche ou structure, par exemple.

L'une au moins des première et deuxième couches ou structures peut présenter une aimantation permanente.

La deuxième couche ou structure peut être une deuxième couche de produit comportant des corps magnétiques dispersés et l'interaction magnétique peut contribuer à orienter et/ou à déplacer au moins une partie des corps magnétiques présents dans la deuxième couche, afin par exemple de créer un effet esthétique nouveau, notamment l'apparition d'un motif. Ce motif peut avoir une forme qui peut être liée à celle de la première couche ou structure et/ou à la disposition des lignes de champ magnétique.

Dans des exemples non limitatifs de mise en oeuvre de l'invention, une première couche de produit est appliquée et une deuxième structure est amenée à recouvrir au moins partiellement la première couche de produit. La première couche de produit peut contenir un solvant volatile et la deuxième structure être déposée sur la première couche après séchage de celle-ci. La deuxième structure peut être un faux ongle, un faux tatouage, une mouche, un bijou, un film ou patch ou tout autre article décoratif, notamment poudre, paillettes ou fibres. La deuxième structure peut notamment être souple et, le cas échéant, être découpée à un format souhaité ou selon un motif donné. L'interaction magnétique entre la première couche de produit et la deuxième structure peut contribuer à maintenir la deuxième structure sur la première couche tout en permettant à l'utilisateur d'enlever ou de remplacer facilement la deuxième structure, avantageusement sans endommager la première couche.

Lorsque la première couche est appliquée sur l'ongle, ce dernier présente alors avantageusement l'aspect d'un ongle vernis. Les propriétés magnétiques de la première couche permettent à l'utilisateur de rapporter facilement sur l'ongle un article décoratif, par exemple un faux ongle, et d'en changer selon par exemple sa tenue vestimentaire.

L'amovibilité des faux ongles ou autres articles permet également à l'utilisateur de les enlever temporairement pour effectuer certaines taches, puis de les remettre ensuite, sans craindre une perte d'adhérence.

Dans d'autres exemples non limitatifs de mise en oeuvre de l'invention, une première structure est appliquée et une deuxième couche de produit ou structure est amenée à recouvrir au moins partiellement la première structure. La première structure peut alors être adhésive, par exemple. La première structure peut notamment comporter un film souple, qui peut être adhésif et/ou découpé au format ou selon un motif souhaité. La première structure peut encore être déposée par transfert. Lorsqu'une deuxième couche de produit est appliquée sur la structure, l'interaction magnétique peut modifier par exemple l'orientation de pigments magnétiques contenus dans la deuxième couche et créer un effet esthétique. Les lignes de champ magnétique peuvent avoir une distribution permettant l'apparition de motifs difficiles à réaliser autrement.

Dans des exemples non limitatifs de mise en oeuvre de l'invention, une première couche de produit présentant des propriétés magnétiques est appliquée ainsi qu'une deuxième couche de produit comportant des corps magnétiques orientables sous l'effet d'une interaction magnétique avec la première couche de produit.

La première couche de produit peut être appliquée selon un motif, par exemple manuellement ou au moyen d'une imprimante à jet d'encre ou autrement, par exemple par transfert.

Dans tous les exemples qui précèdent, la première couche ou structure peut présenter une aimantation permanente, afin par exemple d'interagir avec au moins un corps magnétique de la deuxième couche ou structure, qui peut ne pas présenter d'aimantation permanente mais avoir une susceptibilité magnétique non nulle. La deuxième couche ou structure peut encore présenter une aimantation permanente, ce qui peut accroître l'interaction magnétique.

Une couche ou structure présentant une aimantation permanente peut interagir magnétiquement avec au moins un pigment présentant une susceptibilité magnétique non nulle, contenu dans l'autre couche. Cette interaction magnétique peut provoquer la formation d'un motif au sein de la couche comportant le pigment magnétique, suite à un changement d'orientation des particules du pigment lors de leur alignement sur les lignes de champ.

L'invention a encore pour objet, selon un autre de ses aspects, un kit de maquillage de la main, comportant au moins un et mieux au moins cinq articles présentant des propriétés magnétiques et une forme adaptée à leur fixation par attraction magnétique sur l'ongle. Ce ou ces articles sont par exemple des faux ongles.

L'invention a encore pour objet un faux ongle magnétique. Ce faux ongle peut comporter un aimant et/ou des particules magnétiques dispersées ayant une aimantation permanente.

L'invention a encore pour objet, selon un autre de ses aspects, un kit de maquillage comportant :
- au moins un récipient comportant un produit présentant des propriétés magnétiques,
- au moins une structure magnétique capable d'être fixée par attraction magnétique sur un support sur lequel une couche du produit contenu dans le récipient a été déposée.

Le produit présentant des propriétés magnétiques comporte par exemple une dispersion de corps magnétiques tels que par exemple des particules, notamment pigments, présentant une susceptibilité magnétique non nulle.

La structure aimantée peut être un faux ongle, un bijou, une poudre, une mouche, une paillette ou un faux tatouage, entre autres, comportant un aimant permanent ou une dispersion de corps, notamment de particules et/ou fibres magnétiques, présentant une aimantation permanente.

L'invention a encore pour objet un kit de maquillage comportant :
- un premier récipient comportant une première composition contenant des corps comportant du fer métal, notamment du fer doux,
- un second récipient comportant une seconde composition comportant des corps magnétiques, la seconde composition étant apte, lorsqu'elle est appliquée sous forme d'une couche au dessus ou au dessous d'une couche formée par la première composition, à interagir magnétiquement avec la première composition,
   les corps magnétiques de l'une au moins des première et deuxième compositions présentant une aimantation permanente.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 illustre l'application d'une première couche de produit sur l'ongle,
- la figure 2 illustre la mise en place d'un faux ongle magnétique sur la couche de produit ainsi déposée,
- la figure 3 représente de manière schématique un kit pour la mise en oeuvre du procédé illustré aux figures 1 et 2,
- les figures 4 et 5 sont deux sections transversales d'un faux ongle selon divers exemples de mise en oeuvre de l'invention,
- les figures 6 à 9 illustrent d'autres exemples de mise en oeuvre de l'invention,
- les figures 10 et 14 représentent d'autres exemples de kits,
- les figures 11 et 12 illustrent la mise en oeuvre du kit de la figure 10, et
- la figure 13 est une section selon XIII-XIII de la figure 11.

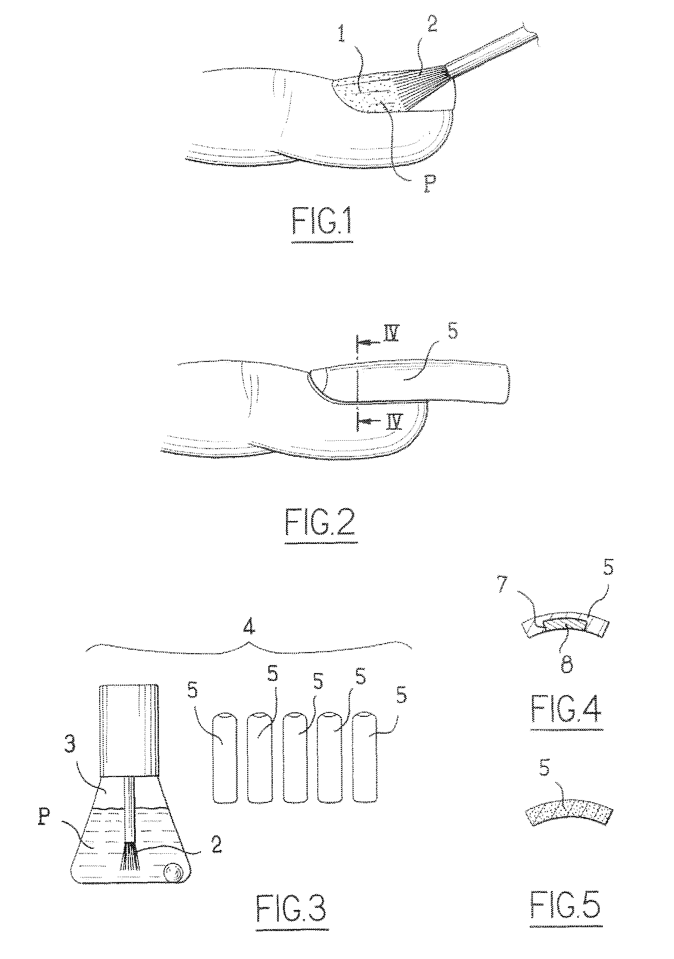

On a illustré à la figure 1 l'application sur l'ongle d'une première couche 1 d'une composition P comportant des corps magnétiques dispersés.

La première couche 1 de composition P est par exemple appliquée sur l'ongle au moyen d'un applicateur 2 tel qu'un pinceau, ce dernier pouvant appartenir à un dispositif de conditionnement et d'application 3 d'un kit 4 représenté à la figure 3.

Une fois la première couche 1 sèche, l'utilisateur peut appliquer sur l'ongle une structure magnétique telle qu'un faux ongle 5, comme illustré à la figure 2. Cette structure est agencée pour interagir magnétiquement avec la première couche 1 et comporte par exemple un aimant permanent 8, comme on peut le voir à la figure 4. Cet aimant 8 est par exemple fixé dans un logement correspondant 7 du faux ongle 5, le corps du faux ongle étant par exemple moulé dans une matière synthétique, par exemple une matière thermoplastique. L'aimant 8 est par exemple collé dans le logement 7.

Dans une variante non illustrée, la matière thermoplastique est surmoulée sur l'aimant.

Le champ magnétique généré par l'aimant 8 est par exemple supérieur ou égal à 100mT.

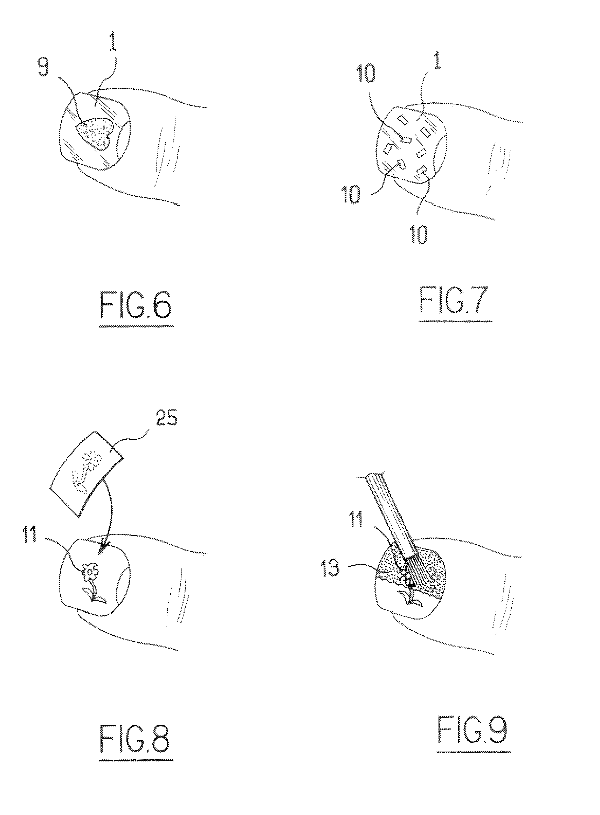

Le faux ongle 5 peut encore, comme illustré à la figure 5, présenter des propriétés magnétiques grâce à l'incorporation dans la matière synthétique thermoplastique ayant servi à le réaliser, d'une charge de particules magnétiques capables de présenter une aimantation permanente.

Plusieurs faux ongles 5, par exemple destinés à l'application sur tous les doigts d'une main, peuvent être proposés à l'utilisateur au sein du kit 4, comme illustré à la figure 3.

Les corps magnétiques dispersés dans la composition P peuvent comporter tout matériau magnétique présentant une sensibilité magnétique non nulle, ce matériau étant par exemple choisi parmi le nickel, le cobalt, le fer, leurs alliages et oxydes, notamment Fe₃O₄, et aussi le gadolinium, le terbium, le dysprosium, l'erbium, Cu₂MnAl, MnBi, leurs alliages et oxydes. Le matériau magnétique peut être de type « doux » ou « dur ». La composition P peut contenir un ferrofluide.

La composition P peut servir uniquement à permettre l'interaction magnétique avec la structure magnétique rapportée dessus, telle que le faux ongle.

La composition P peut encore présenter un aspect lui permettant d'être utilisée, le cas échéant, sans la structure magnétique rapportée.

La composition P peut ainsi avantageusement comporter au moins un agent de coloration, qui peut être magnétique ou non. Cet agent de coloration peut, le cas échéant, procurer à lui seul les propriétés magnétiques de la composition.

Les corps magnétiques dispersés dans la composition peuvent présenter ou non une structure multicouche, comportant au moins une couche d'un matériau magnétique, tel que par exemple le fer, le nickel, le cobalt, leurs alliages et oxydes, notamment Fe₃O₄. Les corps magnétiques peuvent être enrobés, seul le coeur étant par exemple magnétique, l'écorce constituant par exemple une barrière destinée à prévenir de l'oxydation et/ou à conférer de l'innocuité et/ou de la couleur.

L'enrobage est par exemple une matière minérale ou organique, notamment une matière polymère.

La quantité de corps magnétiques est suffisante pour obtenir l'interaction magnétique recherchée. La concentration en corps magnétiques dans la composition P est par exemple comprise entre environ 0,05 et environ 97 % en masse, notamment entre environ 0,1 et environ 95 % en masse, mieux entre environ 0,1 et environ 90 % en masse, par exemple de l'ordre de 3 % en masse.

La dimension des corps magnétiques contenus dans la composition P est par exemple comprise entre 1 nm et 700 µm, mieux entre 1 µm et 500 µm, mieux encore entre 10 µm et 150 µm. Par « dimension », on désigne la dimension donnée par la distribution granulométrique statistique à la moitié de la population, dite D50. Une dimension relativement faible peut faciliter le confort à l'application.

Les corps magnétiques contenus dans la composition P peuvent comporter des pigments magnétiques, comme évoqué plus haut.

Parmi les pigments magnétiques figurent les nacres comportant de l'oxyde de fer Fe₃O₄. Des pigments présentant des propriétés magnétiques sont par exemple ceux commercialisés sous les dénominations commerciales COLORONA BLACKSTAR BLUE, COLORONA BLACKSTAR GREEN, COLORONA BLACKSTAR GOLD, COLORONA BLACKSTAR RED, CLOISONNE NU ANTIQUE SUPER GREEN, MICRONA MATTE BLACK (17437), MICA BLACK (17260), COLORONA PATINA SILVER (17289) et COLORONA PATINA GOLD (117288) de la société MERCK ou bien encore FLAMENCO TWILIGHT RED, FLAMENCO TWILIGHT GREEN, FLAMENCO TWILIGHT GOLD, FLAMENCO TWILIGHT BLUE, TIMICA NU ANTIQUE SILVER 110 AB, TIMICA NU ANTIQUE GOLD 212 GB, TIMICA NU-ANTIQUE COPPER 340 AB, TIMICA NU ANTIQUE BRONZE 240 AB, CLOISONNE NU ANTIQUE GREEN 828 CB, CLOISONNE NU ANTIQUE BLUE 626 CB, GEMTONE MOONSTONE G 004, CLOISONNE NU ANTIQUE RED 424 CB, CHROMA-LITE BLACK (4498), CLOISONNE NU ANTIQUE ROUGE FLAMBE (code 440 XB), CLOISONNE NU ANTIQUE BRONZE (240 XB), CLOISONNE NU ANTIQUE GOLD (222 CB) et CLOISONNE NU ANTIQUE COPPER (340 XB) de la société ENGELHARD.

On peut encore citer les particules d'oxyde de fer noir, par exemple celles commercialisées sous la dénomination SICOVIT noir E172 par la société BASF ou les particules à base de fer doux aciculaire.

La composition P peut comporter au moins un agent de coloration à effets, par exemple un agent de coloration goniochromatique et/ou au moins un pigment diffractant.

Dans un exemple, la composition P peut avoir la formulation suivante, dans le cas d'une application sur l'ongle, les proportions étant exprimées en % en masse.

| | |
|---|---|
| Nitrocellulose | 11 |
| N-éthyl o, p-toluènesulfonamide | 5 |
| Résine alkyde | 10 |
| Isopropanol | 4 |
| | |
| Pigments magnétiques** | 1,5 |
| Acétate de butyle/acétate d'éthyle 50/50 | Qsp 100 |

| | |
|---|---|
| ** Pigments à base de fer doux aciculaire. | |

La structure appliquée sur l'ongle peut être différente d'un faux ongle et se présenter par exemple sous la forme d'un article décoratif 9 tel qu'illustré à la figure 6, cet article pouvant être rigide ou souple.

L'article 9 est par exemple un film, patch ou faux tatouage.

L'article 9 est par exemple découpé dans un film comportant une charge de particules magnétiques dispersées présentant une aimantation permanente. En variante, l'article 9 est réalisé par moulage de matière plastique et comporte un logement recevant un aimant permanent.

L'article 9 peut comporter une pierre naturelle ou de synthèse et former un bijou.

L'article 9 peut encore comporter une impression ou un revêtement décoratif, tel qu'un vernis coloré, par exemple.

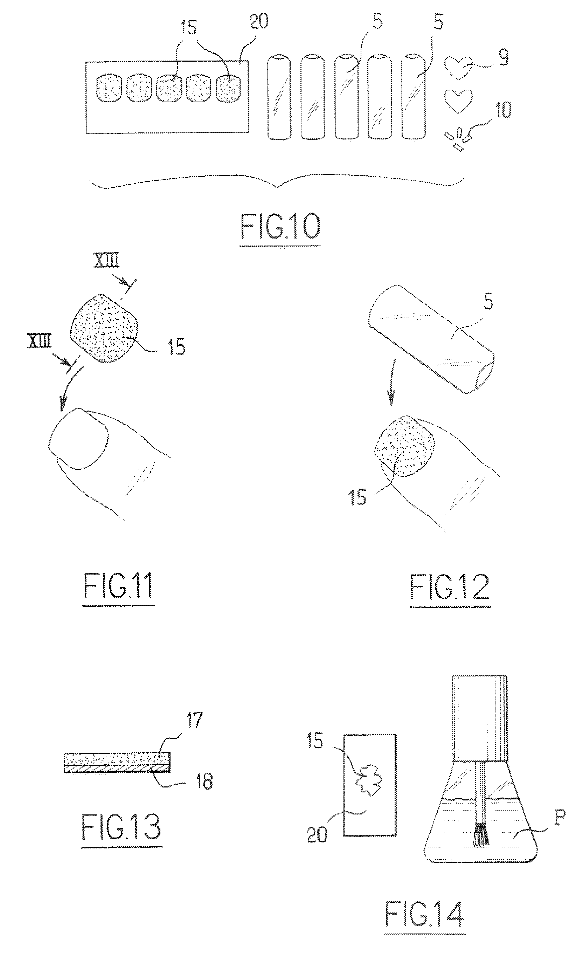

On a illustré à la figure 7 la possibilité d'appliquer sur la première couche de composition P plusieurs éléments décoratifs 10 tels que par exemple des fibres, paillettes ou une poudre. Ces éléments décoratifs 10 peuvent présenter une aimantation permanente. En variante, l'aimantation permanente peut provenir de la première couche 1 de la composition P. Le cas échéant, un vernis de protection peut être appliqué sur le support ainsi décoré. Dans ce cas, ce vernis de protection peut améliorer le maintien du ou des éléments 10 déposés sur l'ongle. L'interaction magnétique entre la première couche et les éléments décoratifs peut contribuer à immobiliser ces derniers avant et lors de l'application du vernis, permettant par exemple à l'utilisateur de les positionner dans la configuration voulue avant l'application du vernis de protection sans craindre leur déplacement lors de l'application de la couche de vernis.

Une première couche d'une composition comportant des corps magnétiques dispersés peut être appliquée, le cas échéant, comme illustré à la figure 8, de manière à former un motif 11 sur le support à maquiller. Cette application peut s'effectuer manuellement, par exemple au moyen d'un pinceau, éventuellement à travers un pochoir, ou à l'aide d'une machine, par exemple une imprimante à jet d'encre telle que décrite par exemple dans US 5 931 166 incorporé par référence, ou encore par transfert, comme illustré. Le motif 11 peut être transféré sous la forme d'un film. Le motif 11 peut être présent sur une bande porteuse 25 avant son transfert.

Ensuite, une deuxième couche 13 d'une composition comportant également des corps magnétiques dispersés, capables d'interagir magnétiquement avec ceux de la première couche, est appliquée sur le support, venant recouvrir le motif 11 et interagissant magnétiquement avec celui-ci. La composition servant à former la deuxième couche 13 présente par exemple la même formulation que la composition P précédemment décrite et contient par exemple des pigments présentant une susceptibilité magnétique non nulle et dont l'orientation est modifiée par l'interaction magnétique avec les corps magnétiques dispersés présents dans le motif 11, lesquels peuvent présenter une aimantation permanente.

La deuxième couche 13 peut par exemple comporter des corps magnétiques asphériques, présentant par exemple une forme allongée. Ainsi, ces corps tendent, lorsque soumis au champ magnétique généré par la première couche ou structure, à s'orienter avec leur axe longitudinal dans l'alignement des lignes de champ, et subissent un changement d'orientation qui se traduit par un changement d'aspect.

Les corps magnétiques peuvent encore présenter un aspect inhomogène, de sorte qu'un changement d'orientation induise un changement d'aspect.

La deuxième couche 13 peut comporter des pigments magnétiques choisis parmi ceux mentionnés précédemment et/ou des fibres magnétiques.

La composition destinée à former la deuxième couche peut être proposée en kit avec au moins une bande porteuse 25 revêtue d'un motif à transférer. En variante, la bande porteuse 25 peut être proposée seule à l'utilisateur.

On a représenté à la figure 10 un autre kit de maquillage comportant au moins une première structure 15 à appliquer sur le support à maquiller, présentant des propriétés magnétiques et au moins une deuxième structure telle que par exemple un faux ongle 5 ou des éléments décoratifs 9 ou 10 destinés à recouvrir la première structure, en interagissant magnétiquement avec celle-ci.

La première structure 15 se présente par exemple sous la forme d'un film souple comportant, comme illustré à la figure 13, une couche d'une matière polymère 17 comportant des corps magnétiques dispersés et une couche d'un adhésif 18 permettant une fixation sur le support à maquiller.

En variante, le film ne présente pas une structure multicouche mais une structure monocouche, et présente des propriétés adhésives liées par exemple à l'évaporation seulement partielle d'un solvant.

La première structure 15 est par exemple destinée à être découpée au format correspondant au support à maquiller, avant sa fixation sur le support ou après celle-ci. Une fois la première structure 15 fixée sur le support, la deuxième structure peut être appliquée sur celle-ci comme illustré à la figure 12 et rester maintenue dessus grâce à une interaction magnétique.

La première structure 15 peut présenter une aimantation permanente de par la présence de corps magnétiques présentant une aimantation permanente dans la couche polymère 17.

Le kit de la figure 10 comporte par exemple autant de premières structures 15 que de doigts à maquiller, et les premières structures 15 sont par exemple fixées sur une feuille de support 20 anti-adhésive pour être facilement décollée en vue de la fixation sur le support à maquiller.

On a représenté à la figure 14 un autre exemple de kit dans lequel la première structure 15 est prédécoupée pour former un motif. Le kit comporte une composition magnétique P destinée à recouvrir le support à maquiller après application de la première structure sur celui-ci, de manière similaire à ce qui a été décrit en référence aux figures 8 et 9.

L'invention n'est pas limitée aux exemples qui viennent d'être décrits. En particulier, l'invention s'applique non seulement au maquillage des ongles mais également à celui de la peau, des lèvres ou des fibres kératiniques.

La composition P destinée à recouvrir ou à être recouverte, peut contenir l'un au moins des composés suivants, selon notamment la nature du support maquillé.

### Agents de coloration goniochromatiques

Par « agent de coloration goniochromatique », on désigne un agent de coloration permettant d'obtenir, lorsque la composition est étalée sur un support, un trajet de couleur dans le plan a*b* de l'espace colorimétrique CIE 1976 qui correspond à une variation Dh° de l'angle de teinte h° d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la composition a été étalée à l'état fluide avec une épaisseur de 300 µm au moyen d'un étaleur automatique sur une carte de contraste de marque ERICHSEN et de référence Typ 24/5, la mesure étant effectuée sur le fond noir de la carte.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

La structure multicouche peut présenter ou non, par rapport à une couche centrale, une symétrie au niveau de la nature chimique des couches empilées. Selon l'épaisseur et la nature des différentes couches, on obtient différents effets.

Des exemples de structures multicouche interférentielles symétriques sont par exemple les structures suivantes : Fe₂O₃/SiO₂/Fe₂O₃/SiO₂/Fe₂O₃, un pigment ayant cette structure étant commercialisé sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂, des pigments ayant ces structures étant commercialisés sous la dénomination XIRONA par la société MERCK (Darmstadt).

Les agents de coloration à cristaux liquides comprennent par exemple des silicones ou des éthers de cellulose sur lesquels sont greffés des groupes mésomorphes. Comme particules goniochromatiques à cristaux liquides, on peut utiliser par exemple celles vendues par la société CHENIX ainsi que celles commercialisées sous la dénomination HELICONE^{®} HC par la société WACKER.

Comme agent de coloration goniochromatique, on peut encore utiliser certaines nacres, des pigments à effets sur substrat synthétique, notamment substrat type alumine, silice, borosilicate, oxyde de fer, aluminium, ou des paillettes holographiques interférentielles issues d'un film de polytéréphthalate.

La composition P peut en outre comporter des fibres goniochromatiques dispersées. De telles fibres pourront présenter une longueur inférieure à 80 µm par exemple.

La composition P peut également comporter au moins un pigment diffractant, lequel peut présenter des propriétés magnétiques, le cas échéant.

### Pigments diffractants

Par « pigment diffractant », on désigne au sens de la présente invention un pigment capable de produire une variation de couleur selon l'angle d'observation lorsqu'éclairé par de la lumière blanche, en raison de la présence d'une structure qui diffracte la lumière.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel.

On pourra utilement se reporter concernant la structure des pigments diffractants à l'article « Pigments Exhibiting Diffractive Effects » d'Alberto Argoitia and Matt Witzman, 2002, Society of Vacuum coaters, 45th Annual Technical Conference Proceedings 2002.

Le pigment diffractant peut être réalisé avec des motifs ayant différents profils, notamment triangulaires, symétriques ou non, en créneaux, de largeur constante ou non, sinusoïdaux.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette.

Une même particule de pigment peut comporter deux réseaux de diffraction croisés, perpendiculaires ou non.

Le pigment diffractant peut présenter une structure multicouche comportant une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : MgF₂, SiO₂, Al₂O₃, AlF₃, CeF₃, LaF₃, NdF₃, SmF₂, BaF₂, CaF₂, LiF et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr et leurs matériaux, associations ou alliages. Un tel matériau réfléchissant peut, seul, constituer le pigment diffractant qui sera alors monocouche.

En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : MgF₂, SiO, SiO₂, Al₂O₃, TiO₂, WO, AIN, BN, B₄C, WC, TiC, TiN, N₄Si₃, ZnS, des particules de verre, des carbones de type diamant et leurs associations.

En variante, le pigment diffractant peut être composé d'un matériau diélectrique ou céramique préformé tel qu'un minéral en lamelle naturelle, par exemple du mica peroskovite ou du talc, ou des lamelles synthétiques formées à partir de verre, d'alumine, de SiO₂, de carbone, d'un oxyde de fer/mica, de mica recouvert de BN, de BC, de graphite, d'oxychlorure de bismuth, et leurs associations.

A la place d'une couche d'un matériau diélectrique, d'autres matériaux améliorant les propriétés mécaniques peuvent convenir. De tels matériaux peuvent comporter du silicone, des silicides métalliques, des matériaux semi-conducteurs formés à partir d'éléments des groupes III, IV et V, des métaux ayant une structure cristalline cubique centrée, des compositions ou matériaux de cermet, des verres semi-conducteurs, et leurs associations variées.

Le pigment diffractant utilisé peut notamment être choisi parmi ceux décrits dans la demande de brevet américain US 2003/0031870 publiée le 13 février 2003.

Un pigment diffractant peut comporter par exemple la structure suivante : MgF₂/Al/MgF₂, un pigment diffractant ayant cette structure étant commercialisé sous la dénomination SPECTRAFLAIR 1400 Pigment Silver par la société FLEX PRODUCTS, ou SPECTRAFLAIR 1400 Pigment Silver FG. La proportion en poids du MgF₂ peut être comprise entre 80 et 95 % du poids total du pigment.

La quantité de pigment diffractant peut varier, en poids par rapport au poids total de la composition P, par exemple de 0,1 à 5 %.

La dimension du pigment diffractant peut être comprise par exemple entre 5 et 200 µm, mieux entre 5 et 100 µm, par exemple entre 5 et 30 µm.

L'épaisseur des particules de pigment diffractant peut être inférieure ou égale à 3 µm, mieux 2 µm, par exemple de l'ordre de 1 µm.

### Particules réfléchissantes

La composition peut comprendre par exemple des particules réfléchissantes, notamment des paillettes, entre autres, magnétiques ou non.

Par « particules réfléchissantes », on désigne des particules dont la taille, la structure, notamment l'épaisseur de la ou des couches qui la constituent et leurs natures physique et chimique, et l'état de surface, leur permettent de réfléchir la lumière incidente. Cette réflexion peut, le cas échéant, posséder une intensité suffisante pour créer à la surface de la composition ou du mélange, lorsque celui-ci est appliqué sur le support à maquiller, des points de surbrillance visibles à l'oeil nu, c'est-à-dire des points plus lumineux qui contrastent avec leur environnement en semblant briller.

Les particules réfléchissantes peuvent être sélectionnées de manière à ne pas altérer significativement l'effet de coloration généré par les agents de coloration qui leur sont associés et plus particulièrement de manière à optimiser cet effet en terme de rendu de couleur. Elles peuvent plus particulièrement posséder une couleur ou un reflet jaune, rose, rouge, bronze, orangé, brun, or et/ou cuivré.

Les particules réfléchissantes peuvent être présentes dans la composition à une teneur allant de 0,5 % à 60 % par rapport au poids total de la composition P, notamment de 1 % à 30 % en poids, en particulier de 3 % à 10 % en poids.

Ces particules peuvent présenter des formes variées, notamment être en forme de plaquettes ou globulaires, en particulier sphériques.

Les particules réfléchissantes, quelle que soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, notamment des oxydes de titane ou de fer obtenus par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou matériau métallique. Le substrat peut être monomatière, multimatériau, organique et/ou inorganique. Plus particulièrement, il peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.

Le matériau réfléchissant peut comporter une couche de métal ou d'un matériau métallique.

Des particules réfléchissantes sont décrites notamment dans les documents JP-A-09188830, JP-A-10158450, JP-A-10158541, JP-A-07258460 et JP-A-05017710.

Toujours à titre d'exemple de particules réfléchissantes comportant un substrat minéral enrobé d'une couche de métal, on peut citer également les particules comportant un substrat de borosilicate enrobé d'argent.

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes quelle que soit leur forme, peuvent également être choisies parmi les particules à substrat synthétique enrobé au moins partiellement d'au moins une couche d'au moins un matériau métallique, notamment un oxyde métallique, choisi par exemple parmi les oxydes de titane, notamment TiO₂, de fer notamment Fe₂O₃, d'étain, de chrome, le sulfate de baryum et les matériaux suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS^{®} par la société ENGELHARD.

La composition selon l'invention peut comprendre au moins une nacre, magnétique ou non.

### Nacres

Par « nacre », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être introduites dans la composition, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

La composition P peut comporter par exemple au moins une charge, magnétique ou non.

### Charges

Par « charge », on désigne des particules de toute forme, insolubles dans le milieu de la composition, quelle que soit la température à laquelle la composition est fabriquée. Une charge peut servir notamment à modifier la rhéologie ou la texture de la composition. La nature et la quantité des particules pourra dépendre des propriétés mécaniques et des textures recherchées.

A titre d'exemple de charges, on peut citer, entre autres, le talc, le mica, la silice, le kaolin, la séricite, les poudres de polyamide, de polyoléfme, par exemple de polyéthylène, de polytétrafluoroéthylène, de polyméthacrylate de méthyle, de polyuréthane, les poudres d'amidon et les billes de résine de silicone.

La composition P peut encore comporter des colorants, pigments organiques ou laques.

### Colorants, pigments organiques et laques

Les colorants peuvent être liposolubles ou hydrosolubles.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave et le bleu de méthylène.

Les colorants peuvent par exemple représenter de 0,1 à 20 % du poids de la composition P, voire de 0,1 à 6 %, lorsque présents.

Les laques ou pigments organiques peuvent être choisis parmi les matériaux ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les matériaux chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

La composition P peut comporter un pigment composite.

### Pigments composites

Le pigment composite peut être composé notamment de particules comportant :
- un noyau inorganique, magnétique ou non,
- au moins un enrobage au moins partiel d'au moins une matière colorante organique.

Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

Le noyau inorganique du pigment composite peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

Le rapport de la plus grande dimension du noyau à sa plus petite dimension peut être compris entre 1 et 50.

Le noyau inorganique peut présenter une dimension comprise entre environ 1 nm et environ 100 nm, voire entre environ 5 nm et environ 75 nm, par exemple entre environ 10 nm et environ 50 nm.

Le noyau inorganique peut être réalisé dans un matériau choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges.

Les oxydes de titane, notamment TiO₂, de fer, notamment Fe₂O₃, de cérium, de zinc et d'aluminium, les silicates, notamment les aluminosilicates et les borosilicates conviennent tout particulièrement.

Le noyau inorganique peut présenter une surface spécifique, mesurée par la méthode BET, comprise par exemple entre environ 1 m²/g et environ 1000 m²/g, mieux entre environ 10 m²/g et environ 600 m²/g, par exemple entre environ 20 m²/g et environ 400 m²/g.

Le noyau inorganique peut être coloré, le cas échéant.

La matière colorante organique peut être telle que définie plus haut.

Le liant du pigment composite peut être de tout type dès lors qu'il permet à la matière colorante organique d'adhérer à la surface du noyau inorganique.

Le liant peut notamment être choisi parmi une liste non limitative comprenant les matériaux siliconés, les matériaux polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

La composition peut comporter une matière colorante photochrome ou un agent photochrome.

### Agents photochromes

D'une manière générale, un agent de coloration photochrome est un agent colorant ayant la propriété de changer de teinte lorsqu'il est éclairé par la lumière ultraviolette et de rétablir sa couleur initiale lorsqu'il n'est plus éclairé par cette lumière ou encore de passer d'un état non coloré à un état coloré et inversement. En d'autres termes, un tel agent présente des teintes différentes selon qu'il est éclairé par de la lumière contenant une certaine quantité de radiations UV comme dans la lumière solaire ou de la lumière artificielle.

On pourra utilement se référer aux exemples d'agents photochromes décrits dans EP 1 410 786.

### Agents thermochromes

On peut par exemple utiliser l'agent thermochrome commercialisé sous la référence KROMAFAST YELLOW 5GX 02- par la société KROMACHEM LTD.

### Autres agents de coloration

La composition P peut encore comporter des composés piézochromes, notamment tribochromes, ou solvatochromes.

### Autres composants

La composition P comporte un milieu physiologiquement acceptable. Par « milieu physiologiquement acceptable », on désigne un milieu non toxique et susceptible d'être appliqué sur la peau, les phanères ou les lèvres d'êtres humains. Le milieu physiologiquement acceptable est généralement adapté à la nature du support sur lequel doit être appliquée la composition ainsi qu'à la forme sous laquelle la composition est conditionnée.

La composition P peut comporter d'autres ingrédients que ceux décrits plus haut, notamment au moins un solvant, une phase grasse, un polymère filmogène et/ou un actif dermatologique ou cosmétique, notamment en fonction de la forme galénique.

### Solvants

La composition P peut comporter au moins un solvant aqueux ou organique, notamment au moins un solvant organique volatil, notamment une huile organique volatile.

Au sens de la présente invention, on entend par « solvant volatil», un solvant, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm Hg), et de préférence allant de 1,3 Pa à 13 000 Pa (0,01 à 100 mm Hg), et préférentiellement allant de 1,3 Pa à 1300 Pa (0,01 à 10 mm Hg).

Lorsque la composition P comprend un ou plusieurs solvants organiques, ces solvants peuvent être présents en une teneur allant de 0,1 % à 99 %, par rapport au poids total de la composition concernée.

D'une manière générale, la quantité de solvant(s), notamment organique(s), dépendra de la nature du support sur lequel la composition est destinée à être appliquée.

La composition P peut comporter au moins un solvant volatil constitué par une huile volatile.

L'huile peut être une huile siliconée ou une huile hydrocarbonée, ou comporter un mélange de telles huiles.

Au sens de la présente invention, on entend par « huile siliconée », une huile comprenant au moins un atome de silicium, et notamment au moins un groupe Si-O.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C8-C16 (appelées aussi isoparaffines) comme l'isododécane (encore appelé 2,2,4,4,6-pentaméthylheptane), l'isodécane, l'isohexadécane, et par exemple les huiles vendues sous les noms commerciaux d'ISOPARS^{®} ou de PERMETHYLS^{®}.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées tels que le nonafluorométhoxybutane ou le perfluorométhylcyclopentane, et leurs mélanges.

La composition peut comprendre entre 0,01 % et 95 % en poids d'huile volatile, par rapport au poids total de la composition, mieux entre 1 % et 75 % en poids.

La composition P peut comporter au moins un solvant organique choisi dans la liste suivante :
- les cétones liquides à température ambiante, tels que le méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les alcanes liquides à température ambiante tels que le décane, l'heptane, le dodécane, le cyclohexane.

La composition P peut aussi comprendre de l'eau ou un mélange d'eau et de solvants organiques hydrophiles couramment utilisés en cosmétique comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La composition P peut, en outre, contenir des éthers en C₂ et des aldéhydes en C₂-C₄ hydrophiles. L'eau ou le mélange d'eau et de solvants organiques hydrophiles peut être présent dans la composition en une teneur allant par exemple de 0 % à 90 %, notamment 0,1 % à 90 % en poids et de préférence de 0 % à 60 % en poids, notamment 0,1 % à 60 % en poids, par rapport au poids total de la composition.

### Phase grasse

La composition P, par exemple lorsqu'elle est destinée à être appliquée sur les lèvres, peut comporter une phase grasse et notamment au moins un corps gras liquide à température ambiante (25 °C) et/ou un corps gras solide à température ambiante tel que les cires, les corps gras pâteux, les gommes et leurs mélanges. La phase grasse peut, en outre, contenir des solvants organiques lipophiles.

La composition P peut présenter par exemple une phase grasse continue, pouvant contenir moins de 5 % d'eau, notamment moins de 1 % d'eau par rapport à son poids total et en particulier être sous forme anhydre.

Comme corps gras liquides à température ambiante, appelés souvent « huiles », on peut citer : les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité, de lanoline, de lanoline acétylée ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; l'isonanoate d'isononyle, le lanolate d'isopropyle, le trimellilate de tridécyle, le malate de diisostéaryle ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges. Les huiles peuvent être présentes en une teneur allant de 0,01 à 90 %, et mieux de 0,1 à 85 % en poids, par rapport au poids total de la composition.

La présence d'une phase huileuse peut conférer de la brillance et présenter par exemple un indice de réfraction compris entre 1,47 et 1,51, mieux entre 1,48 et 1,50. L'indice de réfraction est mesuré à température ambiante (25 °C), à l'aide d'un réfractomètre.

La composition P peut comporter un corps gras pâteux, une cire ou une gomme.

Les corps gras pâteux sont généralement des composés hydrocarbonés avec un point de fusion compris entre 25 et 60 °C, de préférence entre 30 et 45 °C, et/ou une dureté comprise entre 0,001 et 0,5 MPa, de préférence entre 0,005 et 0,4 MPa, comme les lanolines et leurs dérivés.

Les cires peuvent être solides à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En particulier, les cires peuvent présenter une température de fusion supérieure à 25 °C et mieux supérieure à 45 °C. Les cires peuvent être hydrocarbonées, fluorées et/ou siliconées et être d'origine végétale, minérale, animale et/ou synthétique. Comme cires utilisables on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone. La composition peut contenir de 0 à 50 % en poids de cires, par rapport au poids total de la composition, voire de 1 à 30 % en poids.

Les gommes pouvant être utilisées sont généralement des polydiméthylsiloxanes (PDMS) à haut poids moléculaire ou des gommes de cellulose ou des polysaccharides.

### Polymères filmogènes

La composition P peut encore comporter, par exemple, un polymère filmogène, notamment dans le cas d'un mascara ou d'un vernis à ongles. "Polymère filmogène" désigne un polymère apte à former à lui seul ou en présence d'un agent auxiliaire de filmification, un film continu et adhérent sur un support, notamment sur les matières kératiniques.

Parmi les polymères filmogènes utilisables dans la composition P, on peut citer entre autres les polymères synthétiques, de type radicalaire ou de type polycondensat, les polymères d'origine naturelle, tels que la nitrocellulose ou les esters de cellulose, et leurs mélanges.

Les polymères filmogènes de type radicalaire peuvent être notamment des polymères ou des copolymères vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés α,β-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène.

Parmi les polycondensats filmogènes, on peut citer les polyuréthanes, les polyesters, les polyesters amides, les polyamides, et les polyurées, cette liste n'étant pas limitative.

Les polymères d'origine naturelle, éventuellement modifiés, peuvent être choisis parmi la résine shellac, la gomme de sandaraque, les dammars, les élémis, les copals, les polymères cellulosiques, tels que la nitrocellulose, l'éthylcellulose ou les esters de nitrocellulose choisis, par exemple, parmi l'acétate de cellulose, l'acétobutyrate de cellulose, l'acétopropionate de cellulose, et leurs mélanges.

Le polymère filmogène peut être présent sous la forme de particules solides en dispersion aqueuse ou huileuse, connue généralement sous le nom de latex ou pseudolatex. Le polymère filmogène peut comporter une ou plusieurs dispersions stables de particules de polymères généralement sphériques d'un ou plusieurs polymères, dans une phase grasse liquide physiologiquement acceptable. Ces dispersions sont généralement appelées NAD (Non-Aqueous Dispersion) de polymère par opposition à des latex qui sont des dispersions aqueuses de polymère. Ces dispersions peuvent notamment se présenter sous forme de nanoparticules de polymères en dispersion stable dans ladite phase grasse. Les nanoparticules sont de préférence d'une taille comprise entre 5 et 600 nm. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90^{®}, NEOCRYL A-1070^{®}, NEOCRYL A-1090^{®}, NEOCRYL BT-62^{®,} NEOCRYL A-1079^{®}, NEOCRYL A-523^{®} par la société AVECIA-NEORESINS, DOW LATEX 432^{®} par la société DOW CHEMICAL, DAITOSOL 5000 AD^{®} par la société DAITO KASEI KOGYO; ou encore les dispersions aqueuses de polyuréthane vendues sous les dénominations NEOREZ R-981^{®}, NEOREZ R-974^{®} par la société AVECIA-NEORESINS, les AVALURE UR-405^{®}, AVALURE UR-410^{®}, AVALURE UR-425^{®}, AVALURE UR-450^{®}, SANCURE 875^{®}, SANCURE 861^{®}, SANCURE 878^{®}, SANCURE 2060^{®} par la société GOODRICH, IMPRANIL 85^{®} par la société BAYER, AQUAMERE H-1511^{®} par la société HYDROMER ; les sulfopolyesters vendus sous le nom de marque Eastman AQ par la société Eastman Chemical Products.

La composition P peut comprendre également un agent auxiliaire de filmification favorisant la formation d'un film avec le polymère filmogène.

### Actifs

La composition P peut comporter au moins un actif cosmétique ou dermatologique. Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...), autobronzants. Ces actifs peuvent être utilisés par exemple à des concentrations de 0 à 20 % et notamment de 0,001 à 15 % par rapport au poids total de la composition.

La composition P peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, les colorants ou leurs mélanges.

La composition P peut comprendre, selon le type d'application envisagée, les constituants classiquement utilisés dans les domaines considérés, qui sont présents en une quantité appropriée à la forme galénique souhaitée.

Les caractéristiques des différents exemples peuvent se combiner entre elles au sein de variantes non illustrées.

Le cas échéant, un motif peut être formé par l'application d'un champ magnétique sur une première couche déposée sur le support à maquiller. Cette première couche, après séchage, peut être recouverte de manière amovible par une deuxième couche ou structure se maintenant sur la première grâce à l'interaction magnétique existant avec la première couche.

Dans une variante non illustrée, un mascara magnétique est appliqué sur les cils. Une poudre magnétique est saupoudrée sur les cils. L'attraction magnétique retient les particules sur les cils.

L'expression « comportant un » doit être comprise comme étant synonyme de « comportant au moins un », sauf si le contraire est spécifié.

## Revendications

1. Procédé de maquillage et/ou de décor des matières kératiniques, notamment de la peau, des ongles, ou des cils, le procédé comportant les étapes consistant à:
- i) appliquer sur lesdites matières kératiniques une première couche (1 ; 11) d'une première composition (P) ou une première structure (15) présentant des propriétés magnétiques,
- ii) appliquer sur une portion au moins de la première couche ou de la première structure, une seconde couche d'une seconde composition ou une seconde structure (5 ; 9 ; 10) interagissant magnétiquement avec la première couche ou avec la première structure,
- l'une au moins des première et deuxième structures comportant des corps magnétiques dispersés dans un milieu non magnétique.

2. Procédé selon la revendication 1, dans lequel l'interaction magnétique assure le maintien de la deuxième couche ou deuxième structure sur la première couche ou première structure.

3. Procédé selon la revendication 1 ou 2, dans lequel on applique lors de l'étape ii) une composition (13) comportant des corps magnétiques dispersés dans un milieu, l'interaction magnétique contribuant à orienter et/ou à déplacer au moins une partie des corps magnétiques présents dans la composition (13).

4. Procédé selon la revendication 1, dans lequel on applique en i) une première couche d'une première composition (P) présentant des propriétés magnétiques, et en ii) une structure (5 ; 9 ; 10) recouvrant au moins partiellement la première couche de composition (P), et interagissant magnétiquement avec cette dernière.

5. Procédé selon la revendication 4, dans lequel la première composition contient au moins un solvant volatil, notamment un solvant organique volatil, et dans lequel l'application de la structure en ii) s'effectue après le séchage de la première composition.

6. Procédé selon la revendication 4 ou 5, dans lequel la structure appliquée en ii) est un faux ongle (5).

7. Procédé selon la revendication 4 ou 5, dans lequel la structure appliquée en ii) est un faux tatouage (9), film ou patch, ou comporte une pluralité d'éléments décoratifs (10) tels qu'une poudre, des fibres ou paillettes.

8. Procédé selon la revendication 1, dans lequel on applique en i) une première structure (15) présentant des propriétés magnétiques, et sur tout ou partie de laquelle on forme un dépôt (13) d'une composition, ou on applique une seconde structure, le dépôt (13) et la seconde structure interagissant magnétiquement avec la première structure.

9. Procédé selon la revendication précédente, dans lequel la première structure (15) est adhésive.

10. Procédé selon la revendication 8 ou 9, dans lequel la première structure (15) comporte un film souple.

11. Procédé selon la revendication 1, dans lequel on applique en ii) une deuxième couche d'une composition comportant des corps magnétiques orientables sous l'effet d'une interaction magnétique avec la première couche ou première structure.

12. Procédé selon la revendication 11, dans lequel la première composition contient au moins un solvant volatil, notamment un solvant organique volatil, et dans lequel l'application de la composition en ii) s'effectue après séchage de la première composition.

13. Procédé selon la revendication 11 ou 12, dans lequel la première couche ou structure forme un motif.

14. Procédé selon la revendication 11, dans lequel la première couche est appliquée manuellement ou au moyen d'une imprimante à jet d'encre.

15. Procédé selon la revendication 11, dans lequel la première couche est appliquée par transfert.

16. Procédé selon l'une quelconque des revendications précédentes, dans lequel la première couche ou structure présente une aimantation permanente.

17. Procédé selon l'une quelconque des revendications précédentes, dans lequel la deuxième couche ou structure présente une aimantation permanente.

18. Procédé selon la revendication 1, dans lequel la première composition est un vernis à ongles.

19. Procédé selon la revendication 1, dans lequel la première composition est un mascara.

20. Kit de maquillage de la main comportant au moins un et mieux, au moins cinq articles (5) présentant des propriétés magnétiques et une forme permettant leur fixation par attraction magnétique sur l'ongle.

21. Kit de maquillage comportant :
- au moins un récipient (3) comportant une composition (P) présentant des propriétés magnétiques,
- au moins une structure magnétique (5 ; 9 ; 10) capable d'être fixée par attraction magnétique sur un support sur lequel une couche de la composition contenue dans le récipient a été déposée.

22. Kit selon la revendication précédente, dans lequel la structure magnétique est un faux ongle (5).

23. Kit selon la revendication 21, dans lequel la structure magnétique est une poudre, fibre ou paillette (10).

24. Kit selon la revendication 21, dans lequel la structure magnétique est un film, mouche ou faux tatouage (9).

25. Kit selon la revendication 21, dans lequel la composition comporte au moins un solvant volatil, notamment un solvant organique volatil.

26. Kit selon la revendication 21, dans lequel la composition comporte au moins un polymère filmogène.

27. Kit de maquillage comportant :
- un premier récipient (3) comportant une première composition (P) contenant des corps comportant du fer métal, notamment du fer doux,
- un second récipient comportant une seconde composition comportant des corps magnétiques, la seconde composition étant apte, lorsqu'elle est appliquée sous forme d'une couche au dessus ou au dessous d'une couche formée par la première composition, à interagir magnétiquement avec la première composition,
les corps magnétiques de l'une au moins des première et deuxième composition présentant une aimantation permanente.
